# EUROPÄISCHE PATENTANMELDUNG

(11) **EP 1 978 101 A1**
(43) Veröffentlichungstag der Anmeldung: **08.10.2008**
(21) Anmeldenummer: 08005244.2
(22) Anmeldetag: 20.03.2008
(51) Int. Cl.: C12P 7/62, C12P 7/64

(54) **Verfahren zur Anreicherung mehrfach ungesättigter Fettsäuren**

(30) Priorität: 02.04.2007 EP 07006847
(71) Anmelder: Cognis IP Management GmbH, 40589 Düsseldorf (DE)
(72) Erfinder: Schörken, Ulrich, 40591 Düsseldorf (DE); Kempers, Peter, 41189 Mönchengladbach (DE); Sander, Andreas, 89257 Illertissen (DE)

(57) **Zusammenfassung**

Die vorliegende Erfindung betrifft ein Verfahren zur Anreicherung von PUFA-Acylresten in einem Fettsäureestergemisch, das PUFA-Acylreste und andere Fettsäureacylreste enthält, umfassend das Bereitstellen eines Fettsäureestergemisch, das PUFA-Acylreste und andere Fettsäureacylreste enthält, das in Kontakt bringen dieses Gemisches mit einer Lipase und mit Wasser, wobei das Wasser einen pH-Wert von größer als 7 hat, und wobei das Wasser ein Metallsalz enthält, das mit freien Fettsäuren in Wasser bei einem pH-Wert von größer als 7 schwerlösliche Salze bildet, und wobei die Lipase eine negative Selektivität gegenüber PUFA-Acylresten hat, so dass aus dem Fettsäureestergemisch die anderen Fettsäureacylreste schneller hydrolytisch abgespalten werden als die PUFA-Acylreste, und die Abtrennung des mit PUFA-Acylresten angereicherten, zweiten Fettsäureestergemisches.

## Beschreibung

Die vorliegende Erfindung betrifft ein Verfahren zur Anreicherung von PUFA-Acylresten in einem Fettsäureestergemisch, das PUFA-Acylreste und andere Fettsäureacylreste enthält, umfassend das Bereitstellen eines Fettsäureestergemisch, das PUFA-Acylreste und andere Fettsäureacylreste enthält, das in Kontakt bringen dieses Gemisches mit einer Lipase und mit Wasser, wobei das Wasser einen pH-Wert von größer als 7 hat, und wobei das Wasser ein Metallsalz enthält, das mit freien Fettsäuren in Wasser bei einem pH-Wert von größer als 7 schwerlösliche Salze bildet, und wobei die Lipase eine negative Selektivität gegenüber PUFA-Acylresten hat, so dass aus dem Fettsäureestergemisch die anderen Fettsäureacylreste schneller hydrolytisch abgespalten werden als die PUFA-Acylreste, und die Abtrennung des mit PUFA-Acylresten angereicherten, zweiten Fettsäureestergemisches.

Im Sinne der vorliegenden Erfindung bedeutet Fettsäure eine beliebige gesättigte oder ungesättigte, verzweigte oder unverzweigte aliphatische Carbonsäure. Fettsäuren können gesättigt sein, einfach ungesättigt sein, zweifach ungesättigt sein, oder mindestens dreifach ungesättigt sein.

Fettsäuren als solche werden im Sinne der vorliegenden Erfindung auch als freie Fettsäuren bezeichnet. Im Vergleich dazu bedeutet im Sinne der vorliegenden Erfindung der Begriff Fettsäureacylrest den einbindigen Rest, der durch Entfernung des H-Atoms aus der COOH-Gruppe einer freien Fettsäure erhalten wird. Fettsäureacylreste kommen also zum Beispiel in freien Fettsäuren vor. Sie kommen auch in Estern von Fettsäuren, z. B. Estern mit Glycerin, den so genannten Glyceriden, vor.

Ein Fettsäureglycerid ist ein Ester aus Glycerin und ein, zwei oder drei Fettsäureeinheiten. Ist nur eine OH-Gruppe des Glycerins mit einer Fettsäureeinheit verestert, spricht man von einem Monoglycerid. Sind zwei OH-Gruppen des Glycerins mit jeweils einer Fettsäureeinheit verestert, spricht man von einem Diglycerid. Sind alle drei OH-Gruppen des Glycerins mit jeweils einer Fettsäureeinheit verestert, spricht man von einem Triglycerid.

Im Sinne der vorliegenden Erfindung bedeutet PUFA eine Fettsäure, die mindestens dreifach ungesättigt ist. PUFA leitet sich ab vom englischen polyunsaturated fatty acid.

Eine omega-3-Fettsäure ist eine mindestens dreifach ungesättigte Fettsäure. Sie gehört also zu den genannten PUFA. Eine omega-3-Fettsäure hat eine Doppelbindung zwischen dem dritten und dem vierten C-Atom gerechnet vom Methylende aus, wobei das Methyl-C-Atom als erstes C-Atom gezählt wird. Spezielle omega-3-Fettsäuren sind EPA ((all-Z)-5,8,11,14,17-Eicosapentaensäure) und DHA ((all-Z)-4,7,10,13,16,19-Docosahexaensäure).

PUFA-Glyceride, d. h. Glyceride mit einem hohen Anteil an PUFA-Acylresten an den insgesamt vorhandenen Fettsäureacylresten, werden nach dem Stand der Technik insbesondere nach einem der beiden folgenden Verfahren hergestellt:
1) Umesterung von Fischöl zu Ethylestern, destillative Anreicherung der PUFAs und Resynthese zu Glyceriden. Die Triglyceridsynthese wird dabei zumeist enzymatisch durchgeführt.
2) Selektive Hydrolyse von Fischölen zur Anreicherung der PUFAs in den Glyceriden und destillative Reinigung des PUFA-Glycerids. Die selektive Hydrolyse wird dabei zumeist enzymatisch durchgeführt.

Das Verfahren 1 (die enzymatische Triglyceridsynthese) ist zum Beispiel beschrieben in **EP-A 0 528 844.**

Das Verfahren 2 bzw. die Selektivität von Lipasen gegenüber PUFAs bei der Glyceridhydrolyse ist in mehreren Patentanmeldungen offenbart. Zu nennen sind insbesondere WO 97/19601**,** WO 95/24459**,** WO 96/37586**,** WO 96/37587**,** EP-A 0 741183**,** WO 96/26287**,** WO 00/73254**,** WO 04/043894**,** WO 00/49117 und WO 91/16443.

Aus dem Stand der Technik ist folgendes über Enzymselektivitäten gegenüber PUFAs bekannt. Die meisten Lipasen und Phospholipasen haben eine negative Selektivität gegenüber PUFAs im Vergleich zu anderen Fettsäuren, die typischerweise in Pflanzen- und Fischölen enthalten sind. Mit negativer Selektivität ist gemeint, dass die Lipasen die anderen Fettsäurereste schneller aus Glyceriden hydrolytisch abspalten als die PUFA-Acylreste. Daher verläuft die enzymatische Anreicherung von PUFAs zumeist über eine Veränderung der anderen "nicht-PUFA"-Fettsäuren. Dies kann geschehen durch Veresterung, Umesterung oder Hydrolyse von Estern.

Negative Selektivitäten gegenüber PUFAs sind zum Beispiel beschrieben für Candida und Mucor Lipasen. Einige Enzyme, zum Beispiel solche isoliert aus Kaltwasserfischen, zeigen eine positive Selektivität gegenüber PUFAs

Der Mikropilz Thermomyces lanugenosus wurde früher bezeichnet als Humicola lanuginosa. Aus diesem Grund existieren eine Vielzahl von wissenschaftlichen Abhandlungen zur Lipase aus Thermomyces unter dem Namen Humicola. Die Lipase aus Thermomyces zeichnet sich insbesondere durch ein Aktivitätsoptimum im alkalischen pH-Bereich aus und einer guten Stabilität selbst bei einem pH-Wert von 12-13. Das Enzym wird in Waschmitteln eingesetzt. Die Artikel Biotechnology and Bioengineering 48 (1), 1995; Seiten 78-88 **und** Biotechnology and Bioengineering 48 (3), 1995; Seiten 190-196 geben einen guten Überblick über die Eigenschaften der Thermomyces Lipase.

Lipase aus Thermomyces lanugenosus ist kommerziell erhältlich, zum Beispiel als Flüssigpräparation unter der Marke Lipozym^{®} TL 100 L oder Lipolase^{®} 100 L, erhältlich von der Firma Novozymes A/S, Bagsvaerd, Dänemark.

Auch die übrigen Lipasen, die in den folgenden Beispielen verwendet wurden, sind kommerziell erhältlich. Die Lipase aus Geotrichum candidum wurde selbst hergestellt.

Im folgenden bedeutet U "Unit" und ist eine Aktivitätsangabe für Enzyme.1 U ist die Umsetzung von 1 µmol Substanz pro Minute unter bestimmten, definierten Reaktionsbedingungen.

Die Aktivitätsbestimmung für Lipase aus Thermomyces lanugenosus erfolgt wie folgt: (für Novozymes A/S nach der Vorschrift für Lipozym^{®} und Lipolase^{®}): Es wird die Freisetzung von Buttersäure aus Glycerintributyrat bei 30°C und pH 7 bestimmt. Dazu wird eine 0,16 M Tributyrinlösung eingesetzt und Buttersäure wird unter konstantem pH-Wert mit NaOH titriert. 1 Unit entspricht der Aktivität, die 1 µmol Buttersäure aus Tributyrin pro Minute freisetzt.

Fischöle bestehen im Wesentlichen aus Triglyceriden mit einer Mischung aus gesättigten, einfach und mehrfach ungesättigten Fettsäuren, dabei insbesondere einem hohen Anteil an 5- und 6-fach ungesättigten Fettsäuren, die als gesundheitsfördernder Nahrungsmittelzusatzstoff eingesetzt werden können. Da insbesondere die hochungesättigten Fettsäuren gesundheitsfördernd sind, ist es von Interesse diese anzureichern. Dies kann zum Beispiel durch ein selektives Entfernen der nicht hochungesättigten Fettsäuren aus den Triglyceriden geschehen. Zum Beispiel über eine selektive enzymatische Hydrolyse mit Hilfe von Lipasen.

**Tabelle: Typische Fettsäurezusammensetzungen verschiedener Öle**

| | | | | | | | | | | | |
|---|---|---|---|---|---|---|---|---|---|---|---|
| Typische Zusammensetzung der Hauptbestandteile von Nahrungsmittelfetten (1 = Milchfett; 2 = Schweinetalg, 3 = Rindertalg, 4 = Sonnenblumenöl, 5 = Sojaöl, 6 = Olivenöl, 7 = Rapsöl, 8 = Palmöl, 9 = Sardinenöl, 10 = Thunfischöl, 11 = gehärtetes Pflanzenöl (Sonnenblume)) | | | | | | | | | | | |

| Fettsäure | 1 | 2 | 3 | 4 | 5 | 6 | 7 | 8 | 9 | 10 | 11 |
|---|---|---|---|---|---|---|---|---|---|---|---|
| C4:0 | 3 | | | | | | | | | | |
| C6:0-C10:0 | 5 | | | | | | | | | | |
| C12:0 | 3 | | | | | | | | | | |
| C14:0 | 10 | 3 | 4 | | | | 1 | 1 | 7 | 3 | |
| C16:0 | 33 | 24 | 30 | 4 | 10 | 11 | 4 | 39 | 18 | 21 | < 10 |
| C16:1 | 4 | 3 | 3 | | | | | | 10 | 6 | |
| C18:0 | 9 | 8 | 22 | 4 | 4 | 2 | 1 | 5 | 3 | 6 | > 90 |
| C18:1 | 26 | 46 | 38 | 23 | 22 | 75 | 59 | 45 | 15 | 19 | |
| C18:2 | 2 | 8 | 3 | 64 | 53 | 9 | 22 | 8 | | 2 | |
| C18:3 | | | 1 | | 8 | | 10 | | | 1 | |
| C20:5 | | | | | | | | | 17 | 7 | |
| C22:6 | | | | | | | | | 9 | 24 | |

PUFAs sind durch ihre hohe Zahl an Doppelbindungen sehr temperatur- und oxidationsempfindlich. Es kann leicht zur Ausbildung von Isomeren durch Migration der Doppelbindungen sowie zur Bildung von Peroxiden und Polymeren durch Oxidation kommen. Beim Einsatz von polyungesättigten Fettsäuren im Bereich der Nahrungsergänzungsmittel und Pharmazeutika gilt es solche Nebenprodukte bei der Produktion weitgehend zu vermeiden. Insbesondere bei der Herstellung von hochangereicherten PUFAs aus Fischölen, die besonders interessant sind zum Beispiel zur Verabreichung in Form von Kapseln, kommt es durch thermische Anreicherungsverfahren schnell zu einem Qualitätsverlust des Endproduktes.

Typische, aus dem Stand der Technik bekannte Aufarbeitungsverfahren sind die destillative Fraktionierung zumeist der PUFA-Ethylester, die über chemische Umesterung aus den Fischölen gewonnen werden. Hierbei werden die PUFA-Ester einer hohen thermischen Belastung durch zum Teil mehrmalige Fraktionierung ausgesetzt.
Ein alternatives Verfahren stellt die Harnstoff-Fraktionierung dar. Dieses Verfahren bedeutet zwar keine hohe thermische Belastung, allerdings besteht bei dieser Methode eine große Gefahr der Bildung toxischer Nebenprodukte, die die PUFAs verunreinigen.

Eine alternative Methode zur Anreicherung von PUFAs ist die enzymatische Fraktionierung bei niedriger Temperatur. So lassen sich zum Beispiel PUFAs in Form ihrer Glyceride oder ihrer Ethylester anreichern. Die Anreicherung kann dabei über selektive Hydrolyse, selektive Synthese oder auch selektive Umesterung erfolgen.

Nach der selektiven enzymatischen Reaktion besteht allerdings das Problem der Trennung der verschiedenen Fraktionen (z.B. Fettsäure und Glyceride), die zumeist vollständig ineinander löslich sind. Zumeist werden die Reaktionsgemische aus der enzymatischen Anreicherung in einem zweiten Schritt destillativ getrennt. Auch diese Trennung bedeutet eine thermische Belastung für die PUFAs mit der Gefahr eines Qualitätsverlustes.

Die europäische Patentanmeldung mit der Anmeldenummer 06023997 offenbart ein einstufiges Verfahren zur Herstellung von Zinkricinoleat, wobei Ricinusöl in Gegenwart geeigneter Enzyme, vorzugsweise von Lipasen gespalten und gleichzeitig mit Zinkoxid in wässriger Lösung zum Zinkricinoleat umgesetzt wird.

WO 2005/007864 offenbart ein Verfahren zur Herstellung von Carbonsäuresalzen, wobei insbesondere offenbart wird ein Verfahren zur enzymatischen Hydrolyse von Fischölen im Alkalischen in Gegenwart von Calciumsalzen, wobei die Calciumsalze der in den Fischölen enthaltenen PUFAs erhalten werden.

WO 2005/007864 offenbart, dass das am besten geeignete Enzym für eine alkalische Hydrolyse von Estern Lipase aus Thermomyces lanugenosus ist. Diese Lipase wurde speziell für Waschmittelanwendungen entwickelt und weist eine hohe Aktivität bei alkalischem pH-Wert auf. Die Lipase ist in der Lage in Anwesenheit von z.B. Ca(OH)2 oder Mg(OH)2 aus Estern in einer einstufigen Reaktion Seifen (also Metallsalze) zu erzeugen. Die Seifen können dann über Filtration aus der Reaktionslösung isoliert werden.

WO 07/119811 discloses a method for production of a condensed PUFA oil, which comprises performing an alcoholysis reaction of a PUFA-containing oil-and-fat with a lipase in the presence of at least one compound selected from magnesium oxide, magnesium hydroxide, calcium oxide and calcium hydroxide and a small quantity of water and separating a glyceride fraction.

Prinzipiell sind die meisten Enzyme in der Lage zumindestens für kurze Zeit eine Esterhydrolyse unter alkalischen Bedingungen zu katalysieren. Thermomyces Lipase weist von allen kommerziell erhältlichen Lipasen das beste Preis-Leistungs-Verhältnis auf.

Thermomyces Lipase besitzt wie viele andere Lipasen eine negative Selektivität gegenüber PUFAs auf. Dies ist jedoch nicht aus dem Stand der Technik bekannt, sondern wurde erst im Laufe der Untersuchungen entdeckt, die der vorliegenden Erfindung zu Grunde liegen. Die Anwendung der alkalischen Hydrolyse zur PUFA Anreicherung ist im Stand der Technik nicht beschrieben.

Der vorliegenden Erfindung liegt die Aufgabe zu Grunde ein Verfahren bereit zu stellen, das es erlaubt, in einem Gemisch von Fettsäureglyceriden, das PUFA-Acylreste enthält, diese PUFA-Acylreste anzureichern, ohne dass diese thermisch belastet werden.

Diese Aufgabe wird gelöst durch das folgende erfindungsgemäße Verfahren, das Gegenstand der vorliegenden Erfindung ist:
Ein Verfahren zur Anreicherung von PUFA-Acylresten in einem ersten Fettsäureestergemisch, das PUFA-Acylreste und andere Fettsäureacylreste enthält, umfassend
   - das Bereitstellen des ersten Fettsäureestergemisches, das PUFA-Acylreste und andere Fettsäureacylreste enthält,
   - das in Kontakt Bringen dieses Gemisches mit einer Lipase und mit Wasser,
wobei das Wasser einen pH-Wert von größer als 7 hat,
und wobei das Wasser ein Metallsalz enthält, das mit freien Fettsäuren in Wasser bei einem pH-Wert von größer als 7 schwerlösliche Salze bildet,
und wobei die Lipase eine negative Selektivität gegenüber PUFA-Acylresten hat,
so dass aus dem Fettsäureestergemisch die anderen Fettsäureacylreste schneller hydrolytisch abgespalten werden als die PUFA-Acylreste,
und
- die Abtrennung des mit PUFA-Acylresten angereicherten, zweiten Fettsäureestergemisches (bevorzugt ohne thermischen Aufarbeitungsschritt).

Die folgenden Verfahren sind besondere Ausführungsformen der vorliegenden Erfindung.
- Das erfindungsgemäße Verfahren, wobei das erste Fettsäureestergemisch Fettsäuretriglyceride und optional weiterhin Fettsäurediglyceride und optional weiterhin Fettsäuremonoglyceride enthält und bevorzugt ein Fischöl ist.
- Das erfindungsgemäße Verfahren oder ein bereits genanntes Verfahren, das eine besondere Ausführungsform der vorliegenden Erfindung ist, wobei die Lipase eins solche ist, die bei einem pH-Wert von größer als 7 stabil ist und eine gute Aktivität hat (mindestens 10 %, bevorzugt mindestens 30 %, insbesondere mindestens 50 % der Aktivität, die sie bei einem pH-Wert von unter 7 hat).
- Das erfindungsgemäße Verfahren oder ein bereits genanntes Verfahren, das eine besondere Ausführungsform der vorliegenden Erfindung ist, wobei die Lipase Lipase aus Thermomyces lanugenosus ist.
- Das erfindungsgemäße Verfahren oder ein bereits genanntes Verfahren, das eine besondere Ausführungsform der vorliegenden Erfindung ist, wobei das Metallsalz Calciumhydroxid oder Magnesiumhydroxid, insbesondere Calciumhydroxid, ist. In diesem Fall liegt das Calciumhydroxid oder Magnesiumhydroxid bevorzugt überwiegend als in der wässrigen Phase suspendierter Feststoff vor und nur ein kleiner Anteil des schwerlöslichen Hydroxids ist in Wasser gelöst. Durch das Ausfallen schwerlöslicher Fettsäureseifen (Salze mit Calcium- oder Magnesiumionen) wird das gelöste Calciumhydroxid oder Magnesiumhydroxid verbraucht und durch Auflösen des suspendierten Calciumhydroxid oder Magnesiumhydroxid nachgeliefert.
- Das erfindungsgemäße Verfahren oder ein bereits genanntes Verfahren, das eine besondere Ausführungsform der vorliegenden Erfindung ist, wobei das Abtrennen des mit PUFA-Acylresten angereicherten, zweiten Fettsäureestergemisches umfasst: das Abtrennen der schwerlöslichen Salze der freien Fettsäuren durch Dekantieren oder durch Zentrifugieren.
- Das erfindungsgemäße Verfahren oder ein bereits genanntes Verfahren, das eine besondere Ausführungsform der vorliegenden Erfindung ist, wobei das Abtrennen des mit PUFA-Acylresten angereicherten, zweiten Fettsäureestergemisches umfasst: die Extraktion des mit PUFA-Acylresten angereicherten, zweiten Fettsäureestergemisches, insbesondere mit Kohlenwasserstoffen.

Weiterhin ist ein Gegenstand der vorliegenden Erfindung die Verwendung einer Lipase zur Anreicherung von PUFA-Acylresten in einem ersten Fettsäureestergemisch, das PUFA-Acylreste und andere Fettsäureacylreste enthält, wobei die Anreicherung nach dem erfindungsgemäßen Verfahren oder nach einem Verfahren erfolgt, das eine besondere Ausführungsform der vorliegenden Erfindung ist.

Es wurde insbesondere ein Verfahren entwickelt, bei dem
- die PUFAs selektiv enzymatisch über eine Hydrolyse angereichert werden
- die Hydrolyse alkalisch erfolgt, so dass die freigesetzten nicht PUFA haltigen Fettsäuren als Seifenphase präzipitieren
- die PUFA angereicherten Ester unter schonenden Bedingungen extrahiert werden können

Es wurde insbesondere ein Verfahren entwickelt, bei dem die selektive Hydrolyse direkt in Anwesenheit des Extraktionsmittels durchgeführt wird, so dass nach der Reaktion direkt die angereicherte PUFA-Ester Fraktion isoliert werden kann.

Geeignete Lipasen sind alle Lipasen, die eine negative Selektivität gegenüber PUFAs aufweisen. Bevorzugt sind Lipasen, die eine gute Aktivität unter alkalischen Reaktionsbedingungen haben. Besonders bevorzugt ist Thermomyces lanugenosus Lipase. Der Einsatz der Lipase erfolgt bevorzugt in ihrer freien Form.

Geeignete PUFA-haltige Fettsäureestergemische sind insbesondere: Fischöle, Angereicherte Glyceride, PUFA-Ethylester, bereits konzentrierte PUFA-Ethylester.

Das Verhältnis Wasser zu Öl (w/w) ist frei wählbar, insbesondere 10:1 bis 1:10 (Öl = Fettsäureestergemisch).

Bevorzugte Metallsalze sind zweiwertige Hydroxide, Oxide oder Carbonate. Besonders bevorzugte sind Ca(OH)2 oder Mg(OH)2.

Die Einsatzmenge der Metallsalze, bezogen auf den Nicht-PUFA Anteil im Ester ist bevorzugt 0,3-1,5 Mol zweiwertiges Salz pro 1 Mol Nicht-PUFA Fettsäure gebunden im Ester (entspricht stöchiometrich der 0,6 - 3 fachen Menge).

Die Temperatur, bei der das erfindungsgemäße Verfahren durchgeführt wird, ist 20 - 60°C, besonders 25 - 45 °C.

Das erfindungsgemäße Verfahren wird bevorzugt als Batch-Reaktion gerührt durchgeführt. Am besten unter inerter Atmosphäre, insbesondere bei Normaldruck.

Das Extraktionsmittel kann entweder direkt mit in die Reaktion gegeben werden oder nach der Hydrolyse zur Extraktion eingesetzt werden.
Bevorzugt sind nicht wassermischbare Lösungsmittel wie z.B. Alkane, Ether etc.
Besonders bevorzugt sind Isooktan, Oktan, Heptan, Hexan.

Die Einsatzmenge Extraktionsmittel kann, bezogen auf Ölphase, 1:1 bis 10:1 (w/w) sein.

Die Aufarbeitung kann z.B. erfolgen durch Separation, insbesondere durch Absitzen lassen und Dekantieren, Zentrifugation, Koaleszenzabscheider, mittels eines Separators.

Weitere Aufarbeitungsmöglichkeiten sind die Entfernung des Lösungsmittels unter Vakuum bei Temperaturen < 100 °C, bevorzugt < 80°C.

### Beispiele

### Beispiel 1: Screening nach negativer Selektivität für PUFAs am Beispiel von Makrelenöl

10 g Makrelenöl und 10 g Wasser wurden in einen Kolben gegeben und unter Rühren auf 45 °C bzw. 60 °C aufgeheizt. Verschiedene Enzyme wurden zu den Ansätzen gegeben in einer Menge von maximal 1 % an freiem Enzym (kommerzielle Enzympräparation) bzw. 3 % an immobilisiertem Enzym und die Ansätze wurden unter Rühren inkubiert. Während der Reaktion wurden Proben entnommen und der Umsatz wurde über Säurezahlmessung bestimmt. Ab einem Umsatz von > 40 % Hydrolysegrad wurde die Fettsäurezusammensetzung der freigesetzten Fettsäuren analysiert. Aus diesen Daten wurde rechnerisch der Gehalt an Glycerid gebundenen omega-3 Fettsäuren (darin enthalten hauptsächlich EPA und DHA) bestimmt. Enzyme, die keinen Umsatz von 40 % innerhalb von 24 h Reaktionszeit erreichten, wurden nicht weiter ausgewertet. Der Gehalt an omega-3 Fettsäuren im Ausgangsöl betrug 37,6 %.

**Tabelle: Enzymscreening auf Basis Makrelenöl**

| Enzym | Form | Temperatur | Umsatz | Omega-3 in Glycerid |
|---|---|---|---|---|
| Alcaligenes sp. | frei | 45 °C | 62 % | 51 % |
| Aspergillus niger | frei | 45 °C | < 40 % | |
| Candida antarctica A | frei | 45 °C | 44 % | 52 % |
| Candida antarctica A | immobilisiert | 60 °C | < 40 % | |
| Candida antarctica B | frei | 45 °C | < 40 % | |
| Candida antarctica B | immobilisiert | 60 °C | 43% | 41 % |
| Candida cylindracea | frei | 45 °C | 43 % | 55 % |
| Candida rugosa | frei | 45 °C | 41 % | 55 % |
| Pseudomonas fluorescens | frei | 45 °C | 45 % | 47 % |
| Rhizomucor miehei | immobilisiert | 60 °C | < 40 % | |
| Rhizopus oryzae | frei | 45 °C | 43 % | 43 % |
| Rhizopus niveus | frei | 45 °C | < 40 % | |
| Thermomyces lanugenosus | frei | 45 °C | 61 % | 48 % |
| Thermomyces lanugenosus | immobilisiert | 45 °C | 60 % | 51 % |

Bei einer gleichzeitig guten Hydrolyseaktivität zeigten vor allem die Lipasen aus dem Organismus Candida (Ausnahme Lipase B aus Candida antarctica) und Alcaligenes eine gute negative Selektivität gegenüber PUFAs. Thermomyces lanugenosus Lipase sowohl in freier als auch in immobilisierter Form zeigte ebenfalls eine gute Selektivität bei gleichzeitig guter Hydrolysegeschwindigkeit. Eine leichte negative Selektivität gegenüber PUFAs wurde bei allen getesteten Enzymen beobachtet.

### Beispiel 2: Screening nach negativer Selektivität für PUFAs am Beispiel von Menhadenöl

10 g Menhadenöl und 10 g Wasser wurden in einen Kolben gegeben und unter Rühren auf 45 °C bzw. 60 °C aufgeheizt. Verschiedene Enzyme wurden zu den Ansätzen gegeben in einer Menge von maximal 1 % an freiem Enzym (kommerzielle Enzympräparation) bzw. 3 % an immobilisiertem Enzym und die Ansätze wurden unter Rühren inkubiert. Während der Reaktion wurden Proben entnommen und der Umsatz wurde über Säurezahlmessung bestimmt. Ab einem Umsatz von > 40 % Hydrolysegrad wurde die Fettsäurezusammensetzung der freigesetzten Fettsäuren analysiert. Aus diesen Daten wurde rechnerisch der Gehalt an Glycerid gebundenen omega-3 Fettsäuren (darin enthalten hauptsächlich EPA und DHA) bestimmt. Enzyme, die keinen Umsatz von 40 % innerhalb von 24 h Reaktionszeit erreichten, wurden nicht weiter ausgewertet. Der Gehalt an omega-3 Fettsäuren im Ausgangsöl betrug 38,0 %.

**Tabelle: Enzymscreening auf Basis Menhadenöl**

| Enzym | Form | Temp. | Umsatz | Omega-3 in Glycerid |
|---|---|---|---|---|
| Alcaligenes sp. | frei | 45 °C | 58 % | 53 % |
| Aspergillus niger | frei | 45 °C | < 40 % | |
| Candida antarctica A | frei | 45 °C | 52 % | 59 % |
| Candida antarctica A | immobilisiert | 60 °C | 47% | 56 % |
| Candida antarctica B | frei | 45 °C | < 40 % | |
| Candida antarctica B | immobilisiert | 60 °C | 43 % | 45 % |
| Candida cylindracea | frei | 45 °C | 49 % | 61 % |
| Candida rugosa | frei | 45 °C | < 40 % | |
| Pseudomonas fluorescens | frei | 45 °C | 47 % | 49 % |
| Rhizomucor miehei | immobilisiert | 60 °C | < 40 % | |
| Rhizopus oryzae | frei | 45 °C | < 40 % | |
| Rhizopus niveus | frei | 45 °C | < 40% | |
| Thermomyces lanugenosus | frei | 45 °C | 41 % | 49 % |
| Thermomyces lanugenosus | immobilisiert | 45 °C | 42 % | 50 % |

Bei einer gleichzeitig guten Hydrolyseaktivität zeigten vor allem die Lipasen aus dem Organismus Candida (Ausnahme Lipase B aus Candida antarctica) und Alcaligenes eine gute negative Selektivität gegenüber PUFAs. Thermomyces lanugenosus Lipase sowohl in freier als auch in immobilisierter Form zeigte ebenfalls eine gute Selektivität bei ausreichender Hydrolysegeschwindigkeit. Eine leichte negative Selektivität gegenüber PUFAs wurde bei allen getesteten Enzymen beobachtet.

### Beispiel 3: Screening nach negativer Selektivität für PUFAs am Beispiel von Thunfischöl

10 g Thunfischöl und 10 g Wasser wurden in einen Kolben gegeben und unter Rühren auf 45 °C bzw. 60 °C aufgeheizt. Verschiedene Enzyme wurden zu den Ansätzen gegeben in einer Menge von maximal 1 % an freiem Enzym (kommerzielle Enzympräparation) bzw. 3 % an immobilisiertem Enzym und die Ansätze wurden unter Rühren inkubiert. Während der Reaktion wurden Proben entnommen und der Umsatz wurde über Säurezahlmessung bestimmt. Ab einem Umsatz von > 40 % Hydrolysegrad wurde die Fettsäurezusammensetzung der freigesetzten Fettsäuren analysiert. Aus diesen Daten wurde rechnerisch der Gehalt an Glycerid gebundenen omega-3 Fettsäuren (darin enthalten hauptsächlich EPA und DHA) bestimmt. Enzyme, die keinen Umsatz von 40 % innerhalb von 24 h Reaktionszeit erreichten, wurden nicht weiter ausgewertet. Der Gehalt an omega-3 Fettsäuren im Ausgangsöl betrug 39,6 %.

**Tabelle: Enzymscreening auf Basis Thunfischöl**

| Enzym | Form | Temp. | Umsatz | Omega-3 in Glycerid |
|---|---|---|---|---|
| Alcaligenes sp. | frei | 45 °C | 58 % | 53 % |
| Aspergillus niger | frei | 45 °C | < 40 % | |
| Candida antarctica A | frei | 45 °C | 63 % | 64 % |
| Candida antarctica A | immobilisiert | 60 °C | < 40 % | |
| Candida antarctica B | frei | 45 °C | < 40 % | |
| Candida antarctica B | immobilisiert | 60 °C | 43 % | 46 % |
| Candida cylindracea | frei | 45 °C | 53 % | 64% |
| Candida rugosa | frei | 45 °C | 41 % | 57% |
| Pseudomonas fluorescens | frei | 45 °C | 46 % | 50 % |
| Rhizomucor miehei | immobilisiert | 60 °C | < 40 % | |
| Rhizopus oryzae | frei | 45 °C | 54% | 49% |
| Rhizopus niveus | frei | 45 °C | < 40% | |
| Thermomyces lanugenosus | frei | 45 °C | 61 % | 54 % |
| Thermomyces lanugenosus | immobilisiert | 45 °C | 55 % | 48 % |

Bei einer gleichzeitig guten Hydrolyseaktivität zeigten vor allem die Lipasen aus dem Organismus Candida (Ausnahme Lipase B aus Candida antarctica) eine gute negative Selektivität gegenüber PUFAs. Thermomyces lanugenosus Lipase sowohl in freier als auch in immobilisierter Form zeigte ebenfalls eine gute Selektivität bei gleichzeitig guter Hydrolysegeschwindigkeit. Eine negative Selektivität gegenüber PUFAs wurde bei allen getesteten Enzymen beobachtet. Diese Selektivität ist bei DHA reichem Thunfischöl stärker ausgeprägt als bei den EPA reichen Fischölen.

### Beispiel 4: Enzymscreening am Beispiel CLA-Methylester-Identifizierung geeigneter Lipasen und Esterasen

In 16 Eppendorf-Cups wurden jeweils 100 mg CLA-Methylester (CLA = konjugierte Linolsäure), 0,9 ml Wasser und 15 mg Mg(OH)2 eingewogen. Die Umsetzungen wurden jeweils durch Zugabe einer Lipase bzw. Esterase, wie in der Tabelle aufgelistet, gestartet. Die Proben wurden bei 30°C auf einem Schüttler inkubiert. Nach 5 h wurden jweils 3 mg Mg(OH)2 und erneut Lipase zugegeben. Die Ansätze wurden für insgesamt 24 h bei 30 °C auf dem Laborschüttler inkubiert. Nach 24 h wurde die Inkubation beendet. Die Ansätze werden komplett ohne Produktabtrennung eingefroren. Alle Ansätze wurden lyophilisiert, die gebildeten Seifen wurden sauer aufgeschlossen und die freigesetzten Fettsäuren werden mit Isooktan extrahiert. Die erhaltenen Fettsäuren wurden nach Sylilierung gaschromatographisch analysiert. Die Umsätze der enzymatischen Hydrolysen wurden über Vergleich der Peakflächen der gebildeten freien Säuren zu noch vorhandenen Mono-, Di- und Triglyceriden bestimmt.

| **Ansatz** | **Lipase aus Organismus** | **Menge** | **Menge nach 5h** | **Mg(OH)₂ nach 5 h** | **Umsatz** |
|---|---|---|---|---|---|
| | | | | | |
| 1 | Thermomyces | 5 µl | 5 µl | 3 mg | 100% |
| 2 | Candida antarctica B | 10 µl | 10 µl | 3 mg | 27% |
| 3 | Rhizomucor miehei | 10 µl | 10 µl | 3 mg | 98% |
| 4 | Candida antarctica A | 10 µl | 10 µl | 3 mg | 26% |
| 5 | Candida cylindracea | 10 mg | 10 mg | 3 mg | 96% |
| 6 | Rhizopus javanicus | 10 mg | 10 mg | 3 mg | 96% |
| 7 | Porcine pancreas | 10 mg | 10 mg | 3 mg | 67% |
| 8 | Aspergillus niger | 10 mg | 10 mg | 3 mg | 9% |
| 9 | Candida rugosa | 10 mg | 10 mg | 3 mg | 71% |
| 10 | Mucor javanicus | 10 mg | 10 mg | 3 mg | 98% |
| 11 | Pseudomonas fluorescens | 10 mg | 10 mg | 3 mg | 100% |
| 12 | Rhizopus oryzae | 10 mg | 10 mg | 3 mg | 96% |
| 13 | Pseudomonas sp. | 10 mg | 10 mg | 3 mg | 85% |
| 14 | Chromobacterium viscosum | 10 mg | 10 mg | 3 mg | 99% |
| 15 | Fusarium oxysporum | 10 mg | 10 mg | 3 mg | 99% |
| 16 | Penicilium camenberti | 10 mg | 10 mg | 3 mg | 40% |

Fazit: Alle Lipasen haben unter den alkalischen Reaktionsbedingungen Aktivität gezeigt. Die meisten der getesteten Lipasen erreichten einen Spaltgrad von > 80 %. Thermomyces lanugenosus Lipase zeigte dabei ein besonders gutes Aktivitäts - Kosten Profil.

### Beispiel 5: Enzymscreening zur Synthese von Zinkricinoleat aus Ricinusöl

In 14 verschließbare Gefäße wurden jeweils 10 g Rizinusöl, 1,35 g Zinkoxid und 50 g Wasser eingewogen. Zu den Ansätzen wurden verschiedene Enzyme nach Tabelle unten zudosiert. Die Ansätze wurden für 24 h auf einem Schüttelinkubator bei 30°C inkubiert. Nach Beendigung der Reaktion wurde eine Probe des gebildeten Zinkrizinoleats entnommen und über Zentrifugation wurde die Wasserphase abgetrennt. Die Proben werden GC-chromatographisch auf den Gehalt an Zinkseifen sowie Glyceriden hin analysiert. Dazu wurden 20 mg der Zinkricinoleatproben mit 1 ml BSTFA / MSTFA [6:1] Gemisch 30 min bei 80°C inkubiert und anschließend auf einer DB5 HT Säule analysiert. Die Auswertung erfolgte über die Peakfläche. Zinkseifen wurden in Form der freien Säuren analysiert. Der Gehalt an Ricinolsäure im eingesetzten Rizinusöl und entsprechend in den gebildeten Zinkseifen betrug dabei etwa 88 %. Die Zinkseifen werden im Folgenden vereinfacht Zinkricinoleat genannt.

| **Ansatz** | **Enzym (Gew% auf Rizinusöl)** | **Hersteller** | **Organismus** |
|---|---|---|---|
| 1 | 1 % Lipolase | Novozymes | Thermomyces lanugenosus |
| 2 | 1 % Novozym 388 | Novozymes | Rhizomucor miehei |
| 3 | 1 % Novozym 525 | Novozymes | Candida antarctica B |
| 4 | 1 % Novozym 868 | Novozymes | Candida antarctica A |
| 5 | 1 % Lipase A | Amano | Aspergillus niger |
| 6 | 1 % Lipomod 34 | Biocatalysts | Candida cylindracea |
| 7 | 1 % Lipopan | Novozymes | Fusarium oxysporum |
| 8 | 0,5 % Lipase GC | Amano | Geotrichum candidum |
| 9 | 1 % Lipase M | Amano | Mucor javanicus |
| 10 | 1 % Lipase R | Amano | Penicilium roquefortii |
| 11 | 1 % Lipase L115P | Biocatalysts | Porcine Pancreas |
| 12 | 1 % Lipase PS | Amano | Pseudomonas sp. |
| 13 | 1 % Lipomod 36 | Biocatalysts | Rhizopus javanicus |
| 14 | 1 % Lipase F-AP 15 | Amano | Rhizopus oryzae |

| **Ansatz** | **Zinkricinoleat** | **Monoglycerid** | **Diglycerid** | **Triglycerid** |
|---|---|---|---|---|
| 1 | 78,1 % | 7,8% | 14,1% | 0% |
| 2 | 63,8% | 28,8 % | 7,4% | 0% |
| 3 | 15,1 % | 0,5% | 5,0 % | 79,4% |
| 4 | 6,5% | 0 % | 10,5 % | 83,0 % |
| 5 | 6,8 % | 0% | 7,2% | 86,0% |
| 6 | 88,0% | 0% | 12,0% | 0% |
| 7 | 58,7% | 33,3% | 8% | 0% |
| 8 | 93,2 % | 0 % | 6,8% | 0 % |
| 9 | 60,4 % | 27,5% | 12,1 % | 0 % |
| 10 | 37,5% | 13,8% | 47,6 % | 1,3 % |
| 11 | 18,2% | 0% | 0% | 81,8% |
| 12 | 5,6 % | 0 % | 8,3% | 86,1 % |
| 13 | 92,1 % | 0% | 2,0% | 5,9% |
| 14 | 90,4% | 0% | 4,4% | 9,6% |

**Fazit:** Alle getesteten Lipasen zeigten eine hydrolytische Reaktion bei Einsatz von Rizinusöl unter alkalischen Bedingungen durch Anwesenheit von ZnO im Reaktionsansatz. Besonders geeignet für eine einstufige Hydrolyse gekoppelt mit Zinkseifenbildung sind aus obigem Screening Lipasen / Esterasen bzw. Phospholipasen aus Thermomyces, Rhizomucor, Candida, isb. cylindracea bzw, rugosa, Fusarium, Geotrichum, Mucor und Rhizopus. Die Liste der getesteten Hydrolasen erhebt dabei keinen Anspruch auf Vollständigkeit.

### Beispiel 6: Screening alkalische Anreicherung von konzentrierten PUFA Ethylestern

In 10 gerührte und verschließbare Gefäße wurden jeweils 1 g PUFA Ethylester, 2 g Isooktan, 1 g Wasser und 40 mg Ca(OH)2 vorgelegt und vermischt. Der eingesetzte PUFA-Ethylester hatte einen Gehalt an 69 % Eicosapentaen- und Docosahexaensäure in Summe. Zu den Ansätzen wurden jeweils 50 mg verschiedener kommerzieller Enzympräparationen aus verschiedenen Organismen zugegeben, wie in der Tabelle aufgelistet. Die Ansätze wurden unter Rühren für 24 h inkubiert. Nach 5 h und 24 h wurden Proben entnommen und über Zentrifugation wird die Isooktanphase von der wässrigen Phase und der festen Phase bestehend aus Calciumseifen abgetrennt. Die Isooktanphase enthielt die nicht umgesetzten Ethylester, die gaschromatographisch analysiert wurden.

**Tabelle : Anreicherung von PUFA Ethylestern**

| **Ansatz** | **Lipase** | **EPA + DHA nach 5 h** | **EPA + DHA nach 24 h** |
|---|---|---|---|
| 1 | Thermomyces lanugenosus | 78,9 % | 77,4 % |
| 2 | Rhizomucor miehei | 65,9 % | 67,1 % |
| 3 | Candida cylindracea | 68,1 % | 66,8 % |
| 4 | Rhizopusjavanicus | 68,6 % | 67,2 % |
| 5 | Porcine Pancreas | 67,8 % | 65,3 % |
| 6 | Candida rugosa | 68 % | 67,2 % |
| 7 | Mucor javanicus | 67,2 % | 66,4 % |
| 8 | Pseudomonas fluorescens | 68,9 % | 67,7 % |
| 9 | Rhizopus oryzae | 67,1 % | 65,9 % |
| 10 | Fusarium oxysporum | 68,6 % | 67,1 % |

**Fazit:** Unter den gewählten Reaktionsbedingungen ist nur Thermomyces Lipase in der Lage einen bereits angereicherten PUFA Ethylester weiter anzureichern.

### Beispiel 7: Alkalische Anreicherung von konzentrierten PUFA Ethylestern mit Thermomyces Lipase

In 2 Reaktionsgefäße wurden jeweils 5 g des angereicherten PUFA Ethylesters aus Beispiel 6, 10 g Isooktan und 0,1 g Ca(OH)2 gegeben. Zu Ansatz 1 wurden 1 g Wasser und zu Ansatz 2 wurden 5 g Wasser gegeben. Die Reaktion wurde jeweils durch Zugabe von 100 µl Thermomyces lanugenosus Lipase (Lipozym TL 100, Novozymes) gestartet. Die Ansätze wurden für 48 h bei Taumtemperatur unter Rühren inkubiert. Nach 24 h und 48 h wurden jeweils Proben entnommen entnommen und über Zentrifugation wurde die Isooktanphase von der wässrigen Phase und der festen Phase bestehend aus Calciumseifen abgetrennt. Die Isooktanphase enthielt die nicht umgesetzten Ethylester, die gaschromatographisch analysiert wurden.

**Tabelle: Anreicherung von PUFA Ethylester mit Thermomyces lanugenosus Lipase**

| **Ansatz** | **EPA + DHA nach 24 h** | **EPA + DHA nach 48 h** |
|---|---|---|
| 1 | 72,5% | 78,9% |
| 2 | 72,5% | 76,9% |

**Fazit:** Es konnte unabhängig von der zugegebenem Wassermenge eine gute Anreicherung des PUFA Ethylesters erzielt werden.

### Beispiel 8: Alkalische Anreicherung von PUFA Ethylestern mit Thermomyces Lipase

In 5 Reaktionsgefäße wurden jeweils 20 g eines PUFA Ethylesters mit einem Gehalt an 36,8 % Eicosapentaen- und Docosahexaensäure in Summe und 1,4 g Ca(OH)2 gegeben. Zu den Ansätzen wurde Isooktan und Wasser in variablen Mengen gegeben, wie in der Tabelle aufgelistet. Die Reaktion wurde jeweils durch Zugabe von 200 µl Thermomyces lanugenosus Lipase (Lipozym TL 100, Novozymes) gestartet. Die Ansätze wurden für 48 h bei Raumtemperatur unter Rühren inkubiert. Nach 5 h, 24 h und 48 h wurden jeweils Proben entnommen und über Zentrifugation wurde die Isooktanphase von der wässrigen Phase und der festen Phase bestehend aus Calciumseifen abgetrennt. Die Isooktanphase enthielt die nicht umgesetzten Ethylester, die gaschromatographisch analysiert wurden.

**Tabelle: Anreicherung von PUFA Ethylester mit Thermomyces lanugenosus Lipase**

| **Ansatz** | **Isooktan** | **Wasser** | **EPA+DHA Nach 5 h** | **EPA+DHA nach 24 h** | **EPA+DHA nach 48 h** |
|---|---|---|---|---|---|
| 1 | 40 g | 2 g | 44,3% | 54,3% | 60,2% |
| 2 | 40 g | 20 g | 50,1% | 63,6% | 64,1% |
| 3 | 40 g | 80 g | 49,4% | 64,9% | 63,4% |
| 4 | 80 g | 2 g | 43,8% | 45,7% | 51,9% |
| 5 | 80 g | 20 g | 48,3% | 65,2% | 66,8% |

**Fazit:** Es wurde in allen Ansätzen eine deutliche Anreicherung der PUFAs (EPA + DHA) in der Ethylesterphase erreicht. Eine Erhöhung des Wassergehaltes im Hydrolyseansatz führte zu einer Erhöhung der Spaltgeschwindigkeit und damit zu einer verbesserten Anreicherung pro Reaktionszeit.

### Beispiel 9: Alkalische Anreicherung von PUFA Ethylester mit Thermomyces Lipase

In 1 Reaktionsgefäße wurden 20 g des PUFA Ethylesters aus Beispiel 8,1,4 g Ca(OH)2 gegeben und 60 g Wasser gegeben. Die Reaktion wurde durch Zugabe von 100 µl Thermomyces lanugenosus Lipase (Lipozym TL 100, Novozymes) gestartet. Nach 6 h Reaktionszeit wurde der Ansatz mit 30 g Isooktan extrahiert. Die Isooktanphase wurde nach 30 min Extraktion von der wässrigen Phase und der festen Phase bestehend aus Calciumseifen über Zentrifugation abgetrennt. Die Isooktanphase enthielt die nicht umgesetzten Ethylester, die gaschromatographisch analysiert wurden.
Die extrahierte Ethylesterphase enthielt 59,1% EPA und DHA in Summe.

## Patentansprüche

1. Ein Verfahren zur Anreicherung von PUFA-Acylresten in einem ersten Fettsäureestergemisch, das PUFA-Acylreste und andere Fettsäureacylreste enthält, umfassend
• das Bereitstellen des ersten Fettsäureestergemisches, das PUFA-Acylreste und andere Fettsäureacylreste enthält,
• das in Kontakt Bringen dieses Gemisches mit einer Lipase und mit Wasser,
wobei das Wasser einen pH-Wert von größer als 7 hat,
und wobei das Wasser ein Metallsalz enthält, das mit freien Fettsäuren in Wasser bei einem pH-Wert von größer als 7 schwerlösliche Salze bildet,
und wobei die Lipase eine negative Selektivität gegenüber PUFA-Acylresten hat, so dass aus dem Fettsäureestergemisch die anderen Fettsäureacylreste schneller hydrolytisch abgespalten werden als die PUFA-Acylreste,
und
• die Abtrennung des mit PUFA-Acylresten angereicherten, zweiten Fettsäureestergemisches.

2. Das Verfahren nach Anspruch 1, wobei das erste Fettsäureestergemisch Fettsäuretriglyceride und optional weiterhin Fettsäurediiglyceride und optional weiterhin Fettsäuremonoglyceride enthält und bevorzugt ein Fischöl ist.

3. Das Verfahren nach Anspruch 1 oder 2, wobei die Lipase eins solche ist, die bei einem pH-Wert von größer als 7 stabil ist und eine gute Aktivität hat (mindestens 10 % der Aktivität, die sie bei einem pH-Wert von unter 7 hat).

4. Das Verfahren nach einem der Ansprüche 1 bis 3, wobei die Lipase Lipase aus Thermomyces lanugenosus ist.

5. Das Verfahren nach einem der Ansprüche 1 bis 4, wobei das Metallsalz Calciumhydroxid oder Magnesiumhydroxid, insbesondere Calciumhydroxid, ist.

6. Das Verfahren nach einem der Ansprüche 1 bis 5, wobei das Abtrennen des mit PUFA-Acylresten angereicherten, zweiten Fettsäureestergemisches umfasst:
• das Abtrennen der schwerlöslichen Salze der freien Fettsäuren durch Dekantieren oder durch Zentrifugieren.

7. Das Verfahren nach einem der Ansprüche 1 bis 6, wobei das Abtrennen des mit PUFA-Acylresten angereicherten, zweiten Fettsäureestergemisches umfasst:
• die Extraktion des mit PUFA-Acylresten angereicherten, zweiten Fettsäureestergemisches.

8. Das Verfahren nach Anspruch 7, wobei die Extraktion mit einem Lösungsmittel erfolgt, das bereits beim in Kontakt Bringen des ersten Fettsäureestergemisches mit einer Lipase und mit Wasser anwesend ist.

9. Die Verwendung einer Lipase zur Anreicherung von PUFA-Acylresten in einem ersten Fettsäureestergemisch, das PUFA-Acylreste und andere Fettsäureacylreste enthält,
wobei die Anreicherung nach einem Verfahren gemäß einem der Ansprüche 1 bis 8 erfolgt.
